**Europäisches Patentamt**

**European Patent Office**

**Office européen des brevets**

(19)

(11) Veröffentlichungsnummer: **0 287 950 B1**

(12) # EUROPÄISCHE PATENTSCHRIFT

(45) Veröffentlichungstag der Patentschrift: **19.11.92**

(51) Int. Cl.⁵: **A61M 5/178**, A61M 5/32

(21) Anmeldenummer: **88105950.5**

(22) Anmeldetag: **14.04.88**

(54) **Sicherheits-Wegwerfspritze.**

(30) Priorität: **24.04.87 IT 6734387**
**10.07.87 IT 6760087**

(43) Veröffentlichungstag der Anmeldung:
**26.10.88 Patentblatt 88/43**

(45) Bekanntmachung des Hinweises auf die
Patenterteilung:
**19.11.92 Patentblatt 92/47**

(84) Benannte Vertragsstaaten:
**AT BE CH DE ES FR GB LI LU NL SE**

(56) Entgegenhaltungen:
**US-A- 3 923 058**
**US-A- 4 026 287**
**US-A- 4 655 751**

(73) Patentinhaber: **Venturini, Aldo**
**Via Don P. Minetti n. 73**
**I-10017 MONTANARO (Torino)(IT)**

(72) Erfinder: **Venturini, Aldo**
**Via Don P. Minetti n. 73**
**I-10017 MONTANARO (Torino)(IT)**

(74) Vertreter: **Aprà, Mario**
**Dipl. Ing. Dr. jur. A. Aprà 27, via Cernaia**
**I-10121 Torino(IT)**

Rank Xerox (UK) Business Services

## Beschreibung

Vorliegende Erfindung betrifft eine Sicherheits-Wegwerfspritze.

Spritzen dieser Art werden beispielsweise zum Einspritzen von Arzneimitteln oder zur Entnahme von Körperflüssigkeiten zu therapeutischen Zwekken oder zur ärztlichen Untersuchung benutzt.

Wegwerfspritzen befinden sich bekanntlich in sterilisiertem und gebrauchsfertigen Zustand im Handel, d.h. sie sind bereits mit der zugehörigen durch eine abnehmbare starre Verschlusskappe oder Hülle geschützten Einspritznadel versehen.

Eine solche Spritze ist aus der US-A-4 655 751 bekannt.

Bei derartigen Spritzen besteht aber die Gefahr, dass die Schutzkappe versehentlich vor dem Gebrauch abgenommen wird, wobei damit das Risiko einerseits der Verseuchung der Einspritznadel und andrerseits einer zufälligen Verletzung bei der Handhabung solcher Spritzen verbunden ist.

Eine noch schwerere Gefahr der Verletzung und Infektion liegt aber bei der Beseitigung derartiger Spritzen nach ihrem Gebrauch vor. Wenn solche Spritzen ohne Schutzkappe oder Hülle für die Einspritznadel weggeworfen werden, bilden sie mögliche Verletzungs- oder Infektionsmittel für Leute, die mit diesen Spritzen nach deren Gebrauch versehentlich und/oder zufällig in Berührung kommen; aber auch das Wiederaufstecken der Schutzkappe auf die Einspritznadel ist mit der Gefahr einer möglichen Verletzung und folglicher Infektion für den Verletzten verbunden.

In jedem Fall stellt die in Gebrauchstellung vorstehende Einspritznadel einer Wegwerfspritze eine enrstliche Verletzungs- und Infektionsgefahr dar für alle jene, die mit einer solchen Spritze in Berührung kommen können.

Der Erfindung liegt die Hauptaufgabe zugrunde,eine Sicherheits-Wegwerfspritze derart auszubilden,dass dabei die oben aufgeführten Nachteile beseitigt sind. Einer weiteren Aufgabe der Erfindung entsprechend soll die erfindungsgemässe Sicherheits-Wegwerfspritze einfach und bequem im Gebrauch sowie sicher und zuverlässig in der Funktion sein. Ausserdem soll die erfindungsgemässe Sicherheits-Wegwerfspritze, falls erwünscht, mit zwei Nadeln bestückt werden können, beispielsweise um mit einer sterilen Nadel Flüssigkeit aus einer Ampulle oder dem menschlichen Körper zu entnehmen und mit der anderen sterilen Nadel die eigentliche Einspritzung vorzunehmen.
Eine solche Sicherheits-Wegwerfspritze soll endlich gestatten, Flüssigkeiten in Ampullen, Sonden od.dgl. einzuspritzen oder daraus zu entnehmen, ohne dass dabei die Einspritznadel, obwohl in der Spritze vorhanden, behinderlich ist.

Den gestellten Aufgaben entsprechend schlägt die Erfindung eine Sicherheits-Wegwerfspritze mit den im kennzeichnenden Teil des Anspruchs 1 angegebenen Merkmalen vor.

Nachfolgend sind Ausführungsbeispiele der Erfindung anhand der beigefügten vereinfschten Zeichnungen näher erläutert. Im Einzelnen seigen:

Fig. 1 eine perspektive Ansicht eines ersten Ausführungsbeispiels der erfindungsgemässen Sicherheits-Wegwerfspritze im auseinandergenommenen Zustand;

Fig. 2 eine schematische Darstellung in einem Axialschnitt der Spritze nach Fig. 1, wie sie für den Gebrauch geboten wird;

Fig. 3 eine Schnittansicht gemäss der Linie III-III in Fig. 2 in grösserem Masstab;

Fig. 4 bis 6 schematische Axialschnitte der Spritze gemäss Fig. 1 bis 3 in aufeinander folgenden Gebrauchsphasen vor dem Wegwerfen der Spritze;

Fig. 7 einen schematischen Axialschnitt der Spritze gemäss Fig. 1 bis 6 zur Darstellung des Wegwerfzustandes der Spritze nach ihrem Gebrauch;

Fig. 8 eine perspektive Ansicht eines weiteren Ausführungsbeispiels der erfindungsgemässen Sicherheits-Wegwerfspritze im auseinander genommenen Zustand;

Fig. 9 eine Teilansicht in Blickrichtung des Pfeils IX in Fig. 8;

Fig.10 eine schematische Darstellung in einem Axialschnitt der Spritze gemäss Fig. 8, wie sie für den Gebrauch geboten wird;

Fig.11 bis 13 schematische Axialschnitte der Spritze gemäss Fig. 8 bis 10 in aifeinander folgenden Gebrauchsphasen vor dem Wegwerfen der Spritze;

Fig.14 einen schematischen Axialschnitt der Spritze gemäss Fig. 8 bis 13 zur Darstellung des Wegwerfzustandes der Spritze nach ihrem Gebrauch.

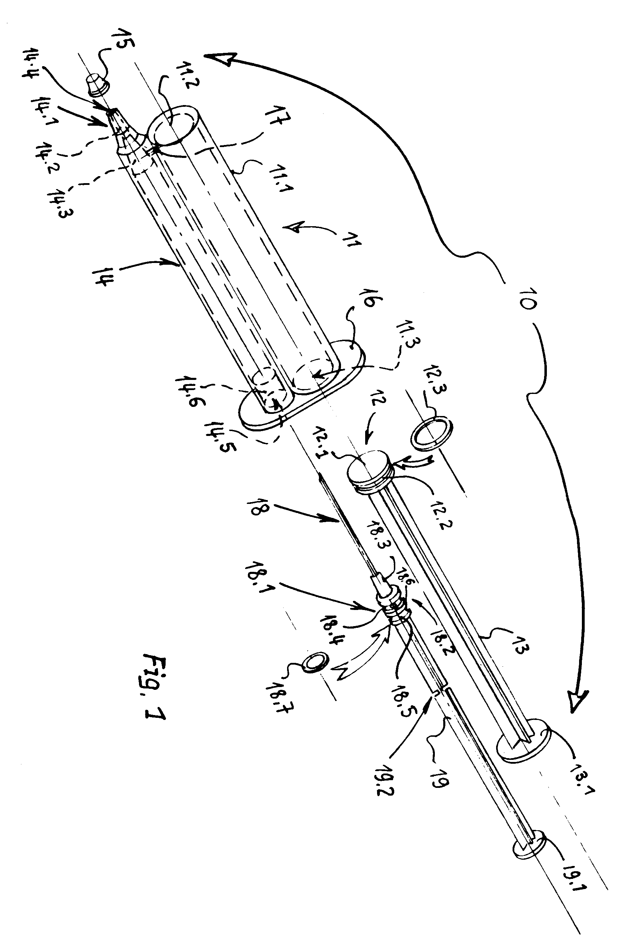

Mit Bezug auf die Figuren 1 bis 7 der Zeichnung ist eine Sicherheits-Wegwerfspritze gemäss einer ersten Ausführungsform der Erfindung in ihrer Gesamtheit mit 10 (Fig. 1 und 2) bezeichnet.

Die Spritze 10 umfasst einen Behälter 11 für einzuspritzende oder abzusaugende Flüssigkeit, der als ein einerends durch eine Bodenwand 11.2 abgeschlossener Hohlzylinder 11.1 ausgebildet ist. Der Behälter 11 ist beispielsweise aus einem geeigneten Kunststoff gefertigt. Im Behälter 11 ist ein scheibenförmiger Kolben 12 mit einem Flachkopf 12.1 verschiebbar angeordnet und mit einem in einer Umfangsnut 12.2 eingesetzten gummielastischen Dichtring 12.3 zur Abdichtung des Kolbens beim Gleiten desselben entlang der zylindrischen Innenwand des Behälters 11 versehen. Am Kolben 12 ist eine starre, koaxial verlaufende und im Querschnitt kreuzförmig ausgebildete Kolbenstange 13 fest angebracht. Am anderen Ende besitzt die Kolbenstange 13 ein Griffteil 13.1, das zur Handbetätigung der Spritze dient, gleichfalls scheibenförmig ausgebildet und am kolbenfernen Ende der Kolbenstange befestigt ist sowie durch eine Öffnung 11.3 des Behälters 11 aus diesem nach aussen vorsteht. Bei diesem ersten dargestellten Ausführungsbeispiel sind Kolben 12, Kolbenstange 13 und Griffteil 13.1 in einem Stück z.B. aus Kunststoff gefertigt.

Ausserdem umfasst die Spritze 10 eine mit dem Behälter 11 einstückig ausgebildete zylinderförmige Rohrhülse 14, die achsparallel zum Hohlzylinder 11.1 des Behälters 11 und tangential an diesem anliegend angeordnet ist. Die Rohrhülse 14 besitzt an einem axialen Ende eine kegelförmig zugespitzte Düse 14.1, die über die Bodenwand 11.2 des Behälters 11 hinaus vorsteht und in genormter Ausführung ausgebildet ist zum lösbaren Aufstecken einer üblichen Einspritznadel mit genormtem Nadelhalter (nicht dargestellt). Im Bereich dieser Düse 14.1 ist in der Rohrhülse 14 koaxial zur selben ein kegelstumpfförmiger Sitz 14.2 ausgebildet; an diesem schliesst sich gegen den Innenraum der Rohrhülse 14 hin eine koaxiale Einmündung 14.3 mit kegeliger Ansenkung und grösserem Kegelwinkel an, wobei diese Einmündung in die zylindrische Innenwand der Rohrhülse 14 übergeht und gegenüber der Bodenwand 11.2 des Behälters 11 um ein Geringes nach hinten zurücksteht. An dem der genannten Einmündung 14.3 abgewandten Ende mündet der kegelstumpfförmige Sitz 14.2 in der Düse 14.1 in eine koaxiale Durchgangsbohrung 14.4. Im normalen Zustand ist auf der Düse 14.1 eine Schutzkappe 15 aufgesteckt.

An ihrem der Düse 14.1 entgegengesetzten Ende besitzt die Rohrhülse 14 eine Öffnung 14.5, die etwa in einer Ebene mit der Öffnung 11.3 des Behälters 11 liegt und in eine versenkte, gegen den Innenraum der Rohrhülse 14 offene und sich an der zylindrischen Innenwand der Rohrhülse anschliessende Einmündung 14.6 übergeht, wobei diese Einmündung gegenüber der angesenkten Einmündung 14.3 des kegelstumpfförmigen Sitzes 14.2 in der Düse 14.1 entgegengesetzt kegelförmig ausgebildet ist.

Im Bereich des Ende des Behälters 11 und der Rohrhülse 14 mit den entsprechenden Öffnungen 11.3 und 14.5 ist am Aussenumfang des Behälters 11 und der Rohrhülse 14 ein in Querrichtung länglicher Flansch 16 als Handhabe für die Spritze 10 einstückig mit diesen beiden Teilen ausgebildet.

Eine radiale Durchgangsbohrung 17 verbindet unter dichtem Abschluss nach Aussen den Behälter 11 mit der Rohrhülse 14 und ist in dem dem Behälter 11 und der Rohrhülse 14 gemeinsamen Wandbereich angeordnet. Diese Durchgangsbohrung 17 befindet sich nahe an der Bodenwand 11.2 des Behälters (Endstellung des Einspritzhubes des Kolbens 12 im Behälter 11) und mündet in die versenkte Einmündung 14.3 des kegelstumpfförmigen Sitzes 14.2 in der Düse 14.1 der Rohrhülse.

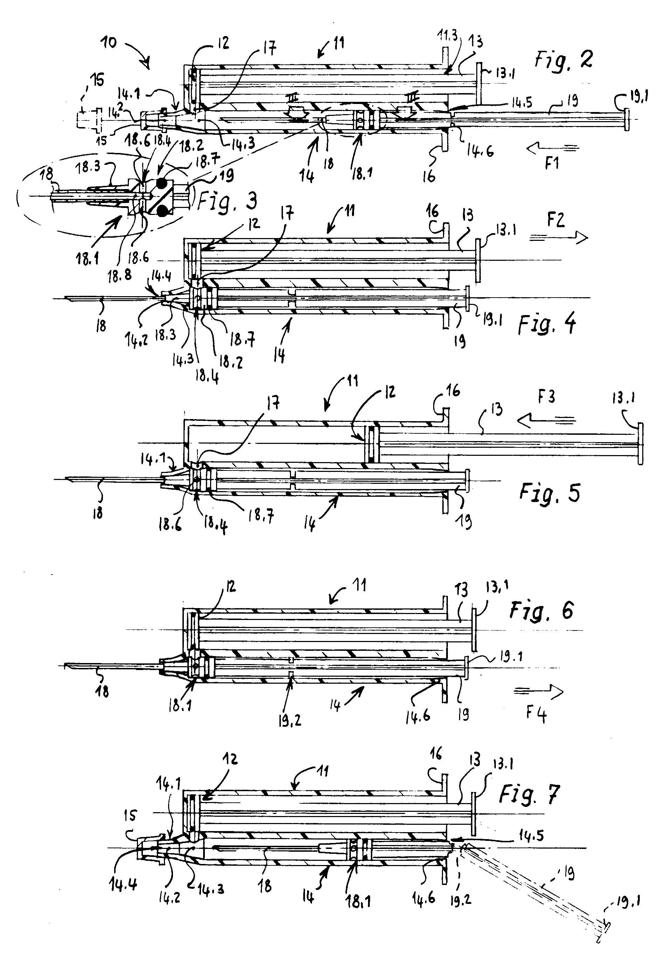

Die Spritze 10 umfasst ausserdem eine Hohlnadel 18, die von einem koaxialen, buchsenförmigen, teilweise hohlen und abgedichtet verschiebbar in der zylindrischen Rohrhülse 14 angeordneten Nadelhalter 18.1 getragen wird, wobei sich die Nadel 18 koaxial und zur Durchgangsbohrung 14.4 der Düse 14.1 hin gerichtet befindet. Der buchsenförmige Nadelhalter 18.1 besteht aus einem im wesentlichen zylindrischen Körper 18.2 und trägt an dem koaxial vorstehenden kegelstumpfförmigen Kopf 18.3 am freien Ende kleineren Durchmessers die Nadel 18. Der zylindrische Körper 18.2 des buchsenförmigen Nadelhalters besitzt an seinem Aussenumfang eine erste Umfangsnut 18.4, die sich nahe der Nadel 18 erstreckt, und eine weitere Umfangsnut 18.5, die von der Nadel 18 weiter entfernt ist. Im Bereich der ersten Umfangsnut 18.4 befinden sich im Nadelhalter zwei radiale, diametral entgegengerichtete Bohrungen 18.6 (Fig. 3), die in den Innenraum des Nadelhalters einmünden, während in der weiteren Umfangsnut 18.5 ein gummielastischer Dichtring 18.7 eingesetzt ist, um ein abgedichtetes Gleiten des Nadelhalters entlang der zylindrischen Innenwand der Rohrhülse 14 sicherzustellen. Wie Fig. 3 zeigt, ist der Innenraum des Nadelhalters 18.1 durch eine axiale Bohrung 18.8 gebildet, die im kegelstumpfförmigen Kopf 18.3 des Nadelhalters zur Lagerung der Nadel 18 dient und eine Verlängerung der Nadelbohrung bildet, wobei sich diese Axialbohrung 18.8 weiter im zylindrischen Körper 18.2 des Halters bis zu den radialen Bohrungen 18.6 erstreckt, die dadurch mit der Axialbohrung 18.8 in Verbindung stehen.

An seinem dem kegelstumpfförmigen Kopf 18.3 entgegengesetzten Ende ist der Nadelhalter 18.1 an einem starren koaxialen Schaft 19 von im dargestellten Ausführungsbeispiel kreuzförmigem Querschnitt befestigt. Der Schaft 19 besitzt an seinem freien aus dem zylindrischen Körper 14 durch die Öffnung 14.5 vorstehenden Ende ein scheibenförmiges Betätigungsteil 19.1. Nadelhalter 18.1, Schaft 19 und Betätigungsteil 19.1 sind im dargestellten Ausführungsbeispiel in einem Stück aus Kunststoff gefertigt.

Ausserdem besitzt der Schaft 19 etwa in der Mitte seiner Länge eine Einschnürung 19.2 seines Querschnittes, die infolge der dadurch verringerten Festigkeit eine Bruchstelle des Schaftes bildet, wie im Folgenden noch ausführlicher Beschrieben wird.

Der buchsenförmige Nadelhalter 18.1 wird bei der Montage durch die Öffnung 14.5 der Rohrhülse 14 bis über die versenkte Einmündung 14.6 der Hülse hinaus eingedrückt.

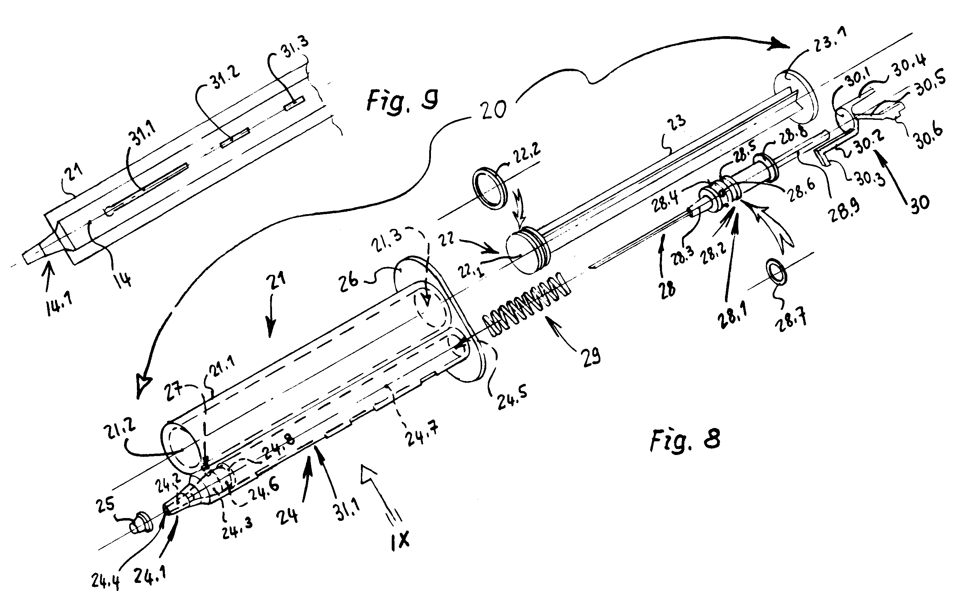

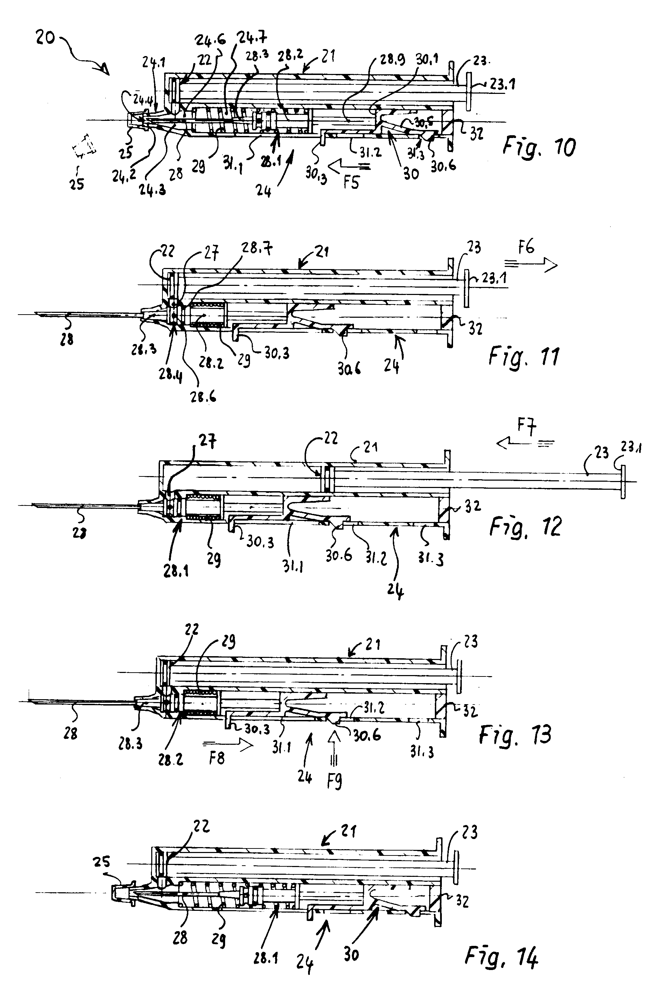

Unter Bezugnahme auf die Fig. 8 bis 14 ist die Sicherheits-Wegwerfspritze gemäss einem weiteren Ausführungsbeispiel der Erfindung in ihrer Gesamtheit mit 20 (Fig. 8, 10) bezeichnet.

Ähnlich wie die Spritze 10 gemäss Fig. 1 bis 7, umfasst die Spritze 20 einen Behälter 21, gebildet durch einen Hohlzylinder 21.1, der einerends durch eine Bodenwand 21.2 abgeschlossen ist. In dem Behälter 21 ist ein dichtfest verschiebbarer scheibenförmiger Kolben 22 mit Flachkopf 22.1 und einem gummielastischen Dichtring 22.2 gelagert. Der Kolben 22 ist fest verbunden mit einer starren koaxialen Kolbenstange 23, deren ebenfalls scheibenförmiges koaxiales Betätigungsteil23.1 am dem Kolben 22 abgewandten Ende der Kolbenstange durch eine Behälteröffnung 21.3 nach aussen vorstehend festgesetzt ist.

Ausserdem besitzt die Spritze 20 in einstückiger Ausführung mit dem Behälter 21 eine zylinderförmige Rohrhülse 24, die achsparallel zum Hohlzylinder 21.1 und an diesem tangential anliegend angeordnet ist. Die Rohrhülse 24 besitzt an einem axialen Ende eine kegelförmig zugespitzte Düse 24.1, die über die Bodenwand 21.2 des Behälters 21 vorsteht. Im Bereich dieser Düse 24.1 ist in der Rohrhülse 24 koaxial dazu ein kegelstumpfförmiger Sitz 24.2 ausgebildet; an diesem schliesst sich gegen den Innenraum der Rohrhülse 24 hin eine koaxiale Einmündung 24.3 mit kegeliger Ansenkung und grösserem Kegelwinkel an, wobei die Einmündung in die zylindrische Innenwand der Rohrhülse 24 übergeht und gegenüber der Bodenwand 21.2 des Behälters etwa nach hinten zurücksteht. An dem der genannten Einmündung 24.3 abgewandten Ende mündet der kegelstumpfförmige Sitz 24.2 in der Düse 24.1 in eine koaxiale, nach aussen hin offene Durchgangsbohrung 24.4. Im normalen Zustand ist auf der Düse 24.1 eine Schutzkappe 25 aufgesteckt. An ihrem der Düse 24.1 entgegengesetzten Ende besitzt die Rohrhülse 24 eine Öffnung 24.5 die im wesentlichen in einer Ebene mit der Öffnung 21.3 des Behälters 21 liegt.

Im Endbereich des Behälters 21 und der Rohrhülse 24 mit den entsprechenden Öffnungen 21.3 und 24.5 ist am Aussenumfang des Behälters und der Rohrhülse ein in Querrichtung länglicher Flansch 26 als Handhabe für die Spritze 20 ausgebildet.

Eine radiale Durchgangsbohrung 27 verbindet unter dichtem Abschluss anch aussen den Behälter 21 mit der Rohrhülse 24 und ist in dem dem Behälter 21 und der Rohrhülse 24 gemeinsamen Wandbereich angeordnet. Diese Durchgangsbohrung 27 befindet sich nahe an der Bodenwand 21.2 des Behälters 21 (Endstellung des Einspritzhubes des Kolbens 22 im Behälter) und mündet in die versenkte Einmündung 24.3 des kegelstumpfförmigen Sitzes 24.2 in der Düse 24.1 der Rohrhülse.

Unterschiedlich gegenüber der Spritze 10 gemäss Fig. 1 bis 7 ist der zylindrische Innenraum der Rohrhülse 24 der Spritze 20 im Querschnitt und weist von der versenkten Einmündung 24.3 des kegelstumpfförmigen Sitzes 24.2 ausgehend in einem ersten kurzen Abschnitt 24.6 einen kleineren Durchmesser auf und in dem zweiten sich am ersten Abschnitt 24.6 anschliessenden und bis zur Öffnung 24.5 der Hülse erstreckenden Abschnitt 24.7 einen grössen Durchmesser. Zwischen diesen beiden Abschnitten 24.6 und 24.7 ist dadurch im zylindrischen Hohlraum der Rohrhülse 24 eine ringförmige Stufe 24.8 gebildet.

Die Spritze 20 umfasst ausserdem eine Hohlnadel 28, die von einem koaxialen, buchsenförmigen, teilweise hohlen und abgedichtet in der zylindrischen Rohrhülse 24 verschiebbar angeordneten Nadelhalter 28.1 getragen wird, wobei sich die Nadel 28 koaxial und zur Durchgangsbohrung 24.4 der Düse 24.1 hin gerichtet befindet. Der buchsenförmige Nadelhalter 28.1 umfasst einen etwa zylinderförmigen Zwischenkörper 28.2 und trägt an dem koaxial vorstehenden kegelstumpfförmigen Kopf 28.3 am freien Ende kleineren Durchmessers die Nadel 28. Der zylindrische Zwischenkörper 28.2 des buchsenförmigen Nadelhalters besitzt an seinem Aussenumfang eine erste Umfangsnut 28.4, die sich nahe der Nadel 28 estreckt, und eine weitere Umfangsnut 28.5, die von der Nadel weiter entfernt ist. Im Bereich der ersten Umfangsnut 28.4 befinden sich im Nadelhalter zwei radiale diametral entgegen gerichtete Bohrungen 28.6 (von denen in den Figuren nur eine sichtbar ist), die in den Innenraum des Nadelhalters einmünden, während in der weiteren Umfangsnut 28.5 ein gummielastischer Dichtring 28.7 eingesetzt ist, um die abgedichtetes Gleiten des Nadelhalters entlang der zylindrischen Innenwand im Bereich des ersten im Durchmesser geringeren Abschnittes 24.6 der Rohrhülse 24 sicherzustellen. Wie bei dem Nadelhalter 18.1 gemäss Fig. 3, ist der(nicht dargestellte) Innenraum des Nadelhalters 28.1 der Spritze 20 durch eine axiale Bohrung gebildet, die im kegelstumpfförmigen Kopf 28.3 des Nadelhalters zur Lagerung der Nadel 28 dient und eine Verlängerung der Nadelbohrung bildet, wobei sich diese Axialbohrung im zylindrischen Zwischenkörper 28.2 des Halters bis zu den Radialbohrungne 28.6 erstreckt, wodurch diese mit der Axialbohrung in Verbindung gesetzt werden.

An seinem vom kegelstumpfförmigen Kopf 28.3 abgewandten Ende besitzt der zylindrische Zwischenkörper 28.2 einen einstückig angeformten Flansch 28.8 grösseren Durchmessers, dessen Umfangsfläche als Gleitfläche bei der Verschiebung in dem Abschnitt 24.7 grösseren Durchmessers des zylindrischen Innenraums der Rohrhülse 24 dient. Gegen die eine Seite des Flansches stützt sich eine Rückholfeder 29 ab, die auf den buchsenförmigen Nadelhalter 28.1 koaxial aufgeschoben ist und deren anderes Ende gegen die erwähnte ringförmige Stufe 24.8 im Innenraum der Rohrhülse 24 abgestützt ist. Von diesem mit dem Flansch 28.8 versehenen Ende des zylindrischen Zwischenkörpers 28.2 des Nadelträgers steht ein kurzer, ebenfalls einstückig angeformter koaxialer Schaft 28.9 vor. Der buchsenförmige Nadelhalter 28.1 ist beispielsweise in einem Stück aus Kunststoff gefertigt.

In zurück versetzter Stellung gegenüber dem Nadelhalter 28.1 ist im Abschnitt 24.7 grösseren Durchmessers des zylindrischen Innenraums der Rohrhülse 24 ein Gleitschuh 30 verstellbar angeordnet und mit einem scheibenförmigen Kopf 30.1 versehen, wobei dieser Kopf zur Führung für die Gleitbewegung im genannten Raum dient und von dem vorne ein Arm 30.2 vorsteht und an der dem Behälter 21 entgegengesetzten Seite der Fläche des erwähnten Innenraums entlang gleitend angeordnet ist. Dieser zum Schaft 28.9 parallel verlaufende Arm besitzt an seinem freien Ende einen Steuerzahn 30.3, der radial durch einen längs verlaufenden Schlitz 31.1 in der Rohrhülse 24 hindurch auf der dem Behälter 21 entgegengesetzten Seite aus der Rohrhülse 24 vorsteht (Fig. 9). Hinter dem scheibenförmigen Kopf 30.1 erstreckt sich ein Abstützfuss 30.4, der auf der Seite des Behälters 21 gegen die Fläche des zylindrischen Innenraums der Rohrhülse 24 gleitend anliegt. Vom mittleren Bereich der Hinterseite des scheibenförmigen Kopfes 30.1 steht ausserdem ein Federblättchen 30.5 in Richtung gegen die Fläche des zylindrischen Innenraums auf der dem Behälter entgegengesetzten Seite vor und trägt an seinem freien Ende eine Rastzunge 30.6, die sich durch wahlweise einen von zwei Längsschlitzen 31.2, 31.3 in der Rohrhülse 24 radial aus der Hülse 24 austreten kann (Fig. 9). Dabei sind die beiden Schlitze unterschiedlich lang (der Schlitz 31.2 ist länger als der Schlitz 31.3) und unter sich sowie zum Führungsschlitz 31.1 gleich ausgerichtet. Die Rastzunge 30.6 ist gegen die Düse 24.1 der Rohrhülse 24 hin nach Art einer Rutsche ausgebildet, um so das Ausrasten von dem einen oder anderen Schlitz bei der Bewegung des Gleitschuhs 30 in Richtung auf die Düse 24.1 hin zu erleichtern, während auf der anderen Seite die Rastzunge im wesentlichen normal zur Achse der Rohrhülse verläuft, um die Raststellung der Zunge gegenüber dem Gleitschuh 30 gegen die Kraft der Rückholfeder 29 sicherzustellen.

Das freie Ende des Schaftes 28.9 des Nadelhalters 28.1 liegt an der Stirnseite des scheibenförmigen Kopfes 30.1 des Gleitschuhs 30 an. Der Gleitschuh 30 ist beispielsweise in einem Stück aus Kunststoff gefertigt.

Die Öffnung 24.5 der Rohrhülse 24 ist mittels eines Stopfens 32 geschlossen (Fig. 10 - 14).

Gebrauch der erfindungsgemässen Spritze 10 (Fig. 2 bis 7):

Die Spritze 10 wird gebrauchsfertig entsprechend der Darstellung von Fig. 2 dargeboten, d.h. mit der in Sicherheitsstellung in der Rohrhülse 14 zurückgezogenen Einspritznadel und mit der auf die Düse 14.1 aufgesteckten Schutzkappe 15 abgeschlossen. Zur Vornahme einer Einspritzung wird die Schutzkappe 15 abgenommen, die Spritze am Flansch 16 ergriffen und dann wird durch Schiebedruck in Axialrichtung (Pfeil F1 in Fig. 2) auf dem Betätigungsteil 19.1 des Schaftes 19 die Nadel 18 durch die Öffnung 14.4 der Düse 14.1 nach aussen verschoben bis der Nadelhalter 18.1 in die Einmündung 14.3 des kegelstupmfförmigen Sitzes 14.2 der Düse eingetreten ist. Durch weiteren Schiebedruck werden dann der kegelstumpfförmige Kopf 18.3 und der zylindrische Körper 18.2 des Nadelhalters in den Sitz 14.2 bzw. in die Einmündung 14.3 gedrückt, womit die Arbeitsstellung der Nadel 18, die nun vollständig aus der Rohrhülse 14 vorsteht, erreicht ist, wobei dadurch in der Rohrhülse 14 ein dicht abgeschlossener Raum zwischen dem kegelstumpfförmigen Kopf 18.3 in dem kegelförmigen Sitz und dem Dichtring 18.7 am Nadelhalter 18.1 durch Anlage des Dichtrings an die zylindrische Innenfläche der Rohrhülse 14 gebildet wird (Fig. 4). Dieser ringförmige, abgedichtete Raum in der Rohrhülse 14 steht nun in dichter Verbindung mit dem Behälter 11 durch die Verbindungsbohrung 17, während in dieser Stellung des Nadelhalters 18.1 dessen Umfangsnut 18.4 - in die die Radialbohrungen 18.6 einmünden - mit der erwähnten Verbindungsbohrung 17 übereinstimmt. Nun wird der Kolben 12 über die Kolbenstange 13 betätigt, um in üblicher Weise eine Arzneiflüssigkeit in den Behälter 11 einzusaugen (Fig. 4, Pfeil F2) und zwar durch die Bohrung der Nadel 18, die Axialbohrung 18.8 und die Radialbohrungen 18.6 des Nadelhalters 18.1, den erwähnten ringförmigen Raum in der Rohrhülse 14 und die Verbindungsbohrung 17; dann wird in umgekehrter Weise und immer bei unveränderter Abdichtung der Spritze die Einspritzung der in den Behälter 11 eingesaugten Flüssigkeit durchgeführt (Fig. 5, Pfeil F3). Nach erfolgter Einspritzung wird die Nadel 18 in die Sicherheits- und Schutzstellung in der Rohrhülse 14 zurückgezogen, indem der Schaft 19 des Nadelhalters 18.1 gezogen wird (Fig. 6, Pfeil F4), bis sich der Schaft 19 mit seiner Bruchstelle 19.2 ausserhalb der Öffnung 14.5 der Rohrhülse 14 befindet. Es wird nun der Schaft 19 abgebrochen (Fig. 7), wobei auf diese Weise die Nadel 18 in der Rohrhülse 14 eingeschlossen bleibt. Dann wird noch die Schutzkappe 15 auf die Düse 14.1 gesteckt. Somit kann die Spritze 10 mit höchster Sicherheit verworfen werden. Dabei ist zu bemerken, dann sich der Nadelhalter 18.1 mit der Nadel 18 keineswegs aus der Rohrhülse nach hinten herausziehen lässt, weil der sich dann in der versenkten Einmündung 14.6 verklemmen würde.

Wenn auch nicht dargestellt, so ist es jedoch einleuchtend, dass das Einsaugen der Flüssigkeit in die Spritze auch dadurch erfolgen kann, dass eine übliche Einspritznadel mit genormtem Nadelträger auf die Düse 14.1 nach Entfernung der Schtzkappe 15 aufgesteckt wird, wobei so die Nadel 18 bislang in geschützter Stellung in der Rohrhülse 14 verbleibt. Nach Entfernung der erwähnten üblichen Nadel kann dann die Nadel 18 herausgetrieben und die Einspritzung vorgenommen werden, wie oben beschrieben wurde.

Ausserdem kann die Spritze 10 bei in die Rohrhülse 14 zurückgezogener Nadel 18 vorteilhaft auch dazu benutzt werden, um beispielsweise Flüssigkeit in Ampullen, Sonden o.dgl. einzuspritzen oder daraus auszusaugen, ohne dass dabei die Nadel irgendwie behinderlich ist.

Mit zurückgezogener Nadel ist die Spritze 10 auch zur Verwendung als Probeglas für Abzapfungen o.dgl. bei Laborarbeiten geeignet.

Gebrauch der erfindungsgemässen Spritzen 20 (Fig. 10 bis 14):

Die Spritze 20 wird gebrauchsfertig wie in Fig. 10 gezeigt dargeboten. Zur Vornahme einer Einspritzung o.dgl. wird die Schutzkappe 25 abgenommen und auf den Gleitschuh 30 über den aus dem Führungsschlitz 31.1 heraustretenden Steuerzahn 30.3 ein Schiebedruck ausgeübt (Fig. 10, Pfeil F5). Dadurch löst sich die Rastzunge 30.6 aus dem Schlitz 31.3 infolge elastischer Biegung des Blättchens 30.5 und rastet in den nächstfolgenden Schlitz 31.2 ein. In diesem Schlitz 31.2 vollführt die Rastzunge 30.6 einen Verstellweg, der der weiteren Verschiebung des Steuerzahnes 30.3 in seinem Führungsschlitz 31.1 entspricht und das Eindrücken des kegelstumpfförmigen Kopfes 28.3 und des zylindrischen Zwischenkörpers 28.2 des Nadelhalters 28.1 in die Einmündung 24.2 bzw. den Sitz 24.3 in der Düse 24.1 der Rohrhülse 24 bewirkt, wobei die Nadel 28 vollständig durch die Durchgangsöffnung 24.4 der Düse hervortritt (Fig. 11). Die Dichtung 28.7 gewährleistet in dieser Stellung die Abdichtung gegenüber der Wand im Bereich 24.6 kleineren Durchmessers des Innenraums der Rohrhülse 24, während in dieser Stellung des Nadelhalters 28.1 sich die Ringnut 28.4 - in die die Radialbohrungen 28.6 einmünden - mit der abgedichteten Verbindungsbohrung 27 zwischen der Rohrhülse 24 und dem Behälter 21 deckt.

So befindet sich nun die Nadel 28 in Arbeitsstellung und ist die Verbindung zwischen der Bohrung der Nadel und dem Behälter 21 in gleichartiger Weise, wie für die Spritze 10 beschrieben, hergestellt. Gleichzeitig wird die Rückholfeder 29 gespannt. In herkömmlicher Weise werden nun durch Verschieben des Kolbens 22 im Behälter 21 die Vorgänge der abgedichteten Einsaugung (Fig. 11, Pfeil F6) und der Einspritzung (Fig. 12, Pfeil F7) vollführt. Um die Nadel in die Sicherheits- und Schutzstellung innerhalb der Rohrhülse 24 zurückzuziehen, wird am Steuerzahn 30.3 gemäss Pfeil F8 in Fig. 13 gezogen und dadurch der Nadelhalter 28.1 aus den Sitzen 24.2 und 24.3 in der Düse 24.1 bzw. in der Rohrhülse 24 entkuppelt; diese Handbetätigung dient dazu, um aus Sicherheitsgründen ein unbeabsichtigten Zurücklaufen der Nadel 28 zu verhindern. Bei dem erwähnten Rückzug greift die Rastzunge 30.6 an der Querseite des Schlitzes 31.2 an. Es genügt dann, die Rastzunge in die Rohrhülse 24 einzudrücken, indem durch Handdruck (Fig. 13, Pfeil F9) die Federspannung des Blättchens 30.5 überwunden wird, damit sich die Wirkung der Rückholfeder 29 entfalten kann und so die Nadel 28 automatisch ihre Sicherheits-und Schutzstellung in der Rohrhülse einnimmt sowie die Rastzunge wieder in den Schlitz 31.3 einrastet. Nach Aufstecken der Schutzkappe 25 kann die Spritze 20 gefahrlos weggeworfen werden.

Es versteht sich, dass an der Spritze gegenüber den beschriebenen und dargestellten Ausführungsbeispielen zahlreiche Änderungen innerhalb des Erfindungsbereichs zulässig sind.

So z.B. kann der Behälter der Spritze eine Vakuum-Kapsel (im Geschäftsverkehr "Vacutainer" genannt) enthalten, oder mit einer elastischen Membran ausgebildet sein, während der Nadelhalter und die Rohrhülse eine andere Formgebung erhalten können, wobei jedoch immer die Funktion der Gleitführung, der stabilen Arbeitsstellung und der Abdichtung der Nadel in der Nadelschutzhülse sicherzustellen sind.

Ausserdem kann die Verbindung zwischen der Nadelbohrung und dem Behälter der Spritze über die Rohrhülse statt durch den Nadelhalter durch die Nadel selbst verwirklicht werden, indem in der Nadelwand Radialbohrungen mit geeigneten Abdichtungsmitteln angebracht werden. In diesem Fall ist die Rohrhülse derart auszubilden, dass sie die verstellbare Nadel unter Abdichtung aufnehmen und deren stabile Arbeitsstellung sicherzustellen vermag, während der Nadelhalter nur die Funktion der Halterung für die Nadel übernimmt.

**Patentansprüche**

1. Sicherheits-Wegwerfspritze (10;20), die einen Behälter (11;21) für einzusaugende und/oder einzuspritzende Flüssigkeiten und eine Rohrhülse (14;24) umfasst, wobei in der Rohrhülse eine Hohlnadel (18;28) mit einem Nadelhalter (18.4;28.4) zwischen einer Sicherheits- und Schutzstellung in der Rohrhülse und einer Arbeitsstellung ausser der Rohrhülse axial hin und zurück verschiebbar ist dadurch gekennzeichnet, dass der Behälter einstückig mit der Rohrhülse ausgebildet ist, wobei ihre Innenräume in Verbindung stehen und wobei sich die Hohlnadel in der erwähnten Arbeitsstellung über die Rohrhülse (14;24) mit dem Behälter (11;21) der Spritze in abgedichteter Verbindung befindet.

2. Spritze nach Anspruch 1, dadurch gekennzeichnet, dass der Nadelhalter (18.1;28.1) mit seinem Endbereich (18.3;28.3), aus dem die Nadel (18;28) vorsteht, in der erwähnten Arbeitsstellung mit aus der Rohrhülse (14;24) ausgetriebener Nadel (18;28) unter leichtem Druck lösbar mit einem in einem axialen Endteil (14.1;24.1) der Rohrhülse eingearbeiteten Sitz (14.2;24.2) einkuppelbar ist und Mittel (18.7;28.7) zur Abdichtung gegenüber der Hohlraumwand der Rohrhülse aufweist, um so eine stabile Arbeitsstellung der Nadel sicherzustellen und gleichzeitig einen abgedichteten Raum in der Rohrhülse zu bilden, sowie um eine dichte Verbindung herzustellen zwischen der Nadelbohrung und dem Behälter (11;21) durch den Nadelhalter hindurch und über eine Verbindungsbohrung (17;27), die in den erwähnten abgedichteten Raum in der Rohrhülse einmündet.

3. Spritze nach Anspruch 2, dadurch gekennzeichnet, dass der erwähnte Sutz (14.2;24.2) in der Rohrhülse (14;24) sich in Richtung auf den Innenraum der Rohrhülse hin erweitert unter Bildung einer Einmündung (14.3;24.3) zum leicht druckweisen und abgedichteten Einkuppeln eines Teiles (18.2;28.2) des Nadelhalters (18.1;28.1), welches Teil sich hinter dem Endbereich (18.3;28.3), von dem die Nadel (18;28) vorspringt, wodurch die Stabilität der Arbeitsstellung der Nadel und die Wirksamkeit der Abdichtung erhöht werden.

4. Spritze nach einem der vorhergehenden Ansprüche, dadurch gekennzeichnet, dass der Nadelhalter (18.1) Dichtmittel (18.7) zur Abdichtung der Gleitbewegung entlang dem gesamten axialen Innenraum der Rohrhülse (14) aufweist, weswegen mittels der Spritze (10) auch in der Sicherheits- und Schutzstellung der Nadel (18) in der Rohrhülse (14) eine Flüssigkeit eingesaugt und/oder eingespritzt werden kann.

5. Spritze nach einem der vorhergehenden Ansprüche, dadurch gekennzeichnet, dass der Nadelhalter (18.1) zusammen mit einem daran befestigten Schaft (19) in der Rohrhülse (14) axial verschiebbar ist, wobei der Schaft von der Rohrhülse nach aussen vorsteht und von Hand betätigbar ist.

6. Spritze nach Anspruch 5, dadurch gekennzeichnet, dass der Schaft (19) eine Bruchstelle (19.2) aufweist, die sich in der Sicherheits- und Schutzstellung der Nadel (18) in der Rohrhülse (14) ausserhalb der Rohrhülse befindet.

7. Spritze nach einem der vorhergehenden Ansprüche 1 bis 5, dadurch gekennzeichnet, dass der Nadelhalter (28.1) bei der axialen Bewegung zum Austreiben der Nadel (28) aus der Rohrhülse (24) mittels eines entgegen der Kraft einer Rückholfeder (29) von Hand betätigbaren Gleitschuhs (30) geführt ist, wobei durch die Rückholfeder nach dem Lösen des Nadelhalters aus der gegenüber der Rohrhülse abgedichteten Einkupplung in Arbeitsstellung der automatische Rücklauf des Nadelhalters samt der Nadel in die Rohrhülse bewirkt wird.

8. Spritze nach einem der vorhergehenden Ansprüche, dadurch gekennzeichnet, dass die Rohrhülse (14;24) in einem Endbereich (14.1;24.1) als Düse ausgebildet ist, wobei auf diese Düse eine herkömmliche Einspritznadel mit normiertem Nadelträger zum Durchführen von Einspritz- und/oder Einsaugvorgängen mit derselben Spritze aufsteckbar ist.

9. Spritze nach einem der vorhergehenden Ansprüche, dadurch gekennzeichnet, dass die Rohrhülse (14) in der Nähe igres axialen Endbereichs (14.5), der dem Endbereich (14.1) mit der Öffnung für den Austritt der Nadel (18) entgegengesetzt ist, eine versenkte Einmündung (14.6) aufweist, die sich in Richtung auf den Innenraum der Rohrhülse erweitert und in der der Nadelhalter (18.1) am Ende seines Rückhubes in der Rohrhülse festgehalten wird.

10. Spritze nach Anspruch 1, dadurch gekennzeichnet, dass die Hohlnadel über wenigstens eine radiale Bohrung, die in der Nadelwand der Einspritznadel vorgesehen ist und in eine mit dem Behälter der Spritze in Verbindung stehende abgedichtete Kammer der Rohrhülse mündet, mit eben demselben Behälter abgedichtet verbindbar ist.

11. Spritze nach einem der vorhergehenden Ansprüche, dadurch gekennzeichnet, dass in dem Behälter (11;21) der Spritze ein Kolben (12;22) mit Betätigungsstange (13;23) abgedichtet verschiebbar angeordnet ist.

12. Spritze nach einem der vorhergehenden Ansprüche 1 bis 10, dadurch gekennzeichnet, dass in dem Behälter eine zum Bewirken eines Saugdruckes in der Spritze bestimmte Vakuum-Kapsel angeordnet ist.

13. Spritze nach einem der vorhergehenden Ansprüche 1 bis 10, dadurch gekennzeichnet, dass der Behälter der Spritze als Kammer mit elastischer Membrane ausgebildet ist.

**Claims**

1. Safety disposable syringe (10; 20) which comprises a container (11; 21) for fluids to be aspirated in and/or injected and a tubular sleeve (14; 24), where a hollow needle (18; 28) with a needle holder (18.4; 28.4) is axially displaceable forwards and backwards in the tubular sleeve between a safety and protecting position in the tubular sleeve and an operating position outside the tubular sleeve, characterised in that the container is designed in one piece with the tubular sleeve, with their interiors being connected, and where the hollow needle in the said operating position is in sealed connection with the container (11; 21) of the syringe via the tubular sleeve (14; 24).

2. Syringe according to Claim 1, characterised in that the needle holder (18.1; 28.1) with its end zone (18.3; 28.3) from which the needle (18; 28) projects can be coupled in the said operating position with needle (18; 28) driven out of the tubular sleeve (14; 24) under light pressure releasably with a seating (14.2; 24.2) incorporated in an axial end part (14.1; 24.1) of the tubular sleeve, and has means (18.7; 28.7) for sealing against the chamber wall of the tubular sleeve in order in this way to ensure a stable operating position of the needle and, at the same time, to form a sealed space in the tubular sleeve, as well as to produce a tight connection between the needle drilling and the container (11; 21) through the needle holder and via a drilled connection (17; 27) which opens into the said sealed space in the tubular sleeve.

3. Syringe according to Claim 2, characterised in that the said seating (14.2; 24.2) in the tubular sleeve (14; 24) expands in the direction towards the interior of the tubular sleeve to form a junction (14.3; 24.3) for the easy pressurewise and sealed coupling in of a part (18.2; 28.2) of the needle holder (18.1; 28.1), which part [lacuna] behind the end zone (18.3; 28.3) from which the needle (18; 28) projects, which increases the stability of the operating position of the needle and the effectiveness of the seal.

4. Syringe according to any of the preceding claims, characterised in that the needle holder (18.1) has sealing means (18.7) for sealing the sliding motion along the entire axial interior of the tubular sleeve (14), for which reason a fluid can be aspirated in and/or injected by means of the syringe (10) even in the safety and protecting position of the needle (18) in the tubular sleeve (14).

5. Syringe according to any of the preceding claims, characterised in that the needle holder (18.1) is axially displaceable together with a shaft (19) affixed thereto in the tubular sleeve (14), with the shaft projecting outwards from the tubular sleeve and being actuable by hand.

6. Syringe according to Claim 5, characterised in that the shaft (19) has a rupture point (19.2) which is located outside the tubular sleeve in the safety and protecting position of the needle (18) in the tubular sleeve (14).

7. Syringe according to any of the preceding Claims 1 to 5, characterised in that the needle holder (28.1) is guided on axial motion to drive out the needle (28) from the tubular sleeve (24) by means of a sliding element (30) which can be actuated by hand against the force of a restoring spring (29), with the restoring spring bringing about the automatic return of the needle holder together with the needle into the tubular sleeve after releasing the needle holder from the coupling in, which is sealed against the tubular sleeve, in the operating position.

8. Syringe according to any of the preceding claims, characterised in that the tubular sleeve (14; 24) is designed in an end zone (14.1; 24.1) as nozzle, it being possible to place on this nozzle a conventional injection needle with standardised needle carrier for carrying out processes of injection and/or aspiration in with the same syringe.

9. Syringe according to any of the preceding claims, characterised in that the tubular sleeve (14) has in the vicinity of its axial end zone (14.5), which is opposite to the end zone (14.1) with the opening for the emergence of the needle (18), a recessed junction (14.6) which expands in the direction towards the interior of the tubular sleeve and in which the needle holder (18.1) is held fast at the end of its return stroke in the tubular sleeve.

10. Syringe according to Claim 1, characterised in that the hollow needle can be connected sealingly via at least one radial drilling which is provided in the needle wall of the injection needle and opens into a sealed chamber, which is connected to the container of the syringe, in the tubular sleeve to exactly the same container.

11. Syringe according to any of the preceding claims, characterised in that a piston (12; 22) with actuation rod (13; 23) is sealingly and displaceably arranged in the container (11; 21) of the syringe.

12. Syringe according to any of the preceding Claims 1 to 10, characterised in that a vacuum capsule intended to bring about a suction pressure in the syringe is arranged in the container.

13. Syringe according to any of the preceding Claims 1 to 10, characterised in that the container of the syringe is designed as chamber with elastic membrane.

**Revendications**

1. Seringue à jeter de sécurité (10; 20) qui comprend un réservoir (11; 21) pour des liquides à aspirer et/ou à injecter et une gaine tubulaire (14; 24), dans laquelle une aiguille creuse (18; 28) avec un porte-aiguille (18.4; 28.4) peut coulisser axialement en aller et retour dans la gaine tubulaire entre une position de sécurité et de protection dans la gaine tubulaire et une position de travail hors de la gaine tubulaire, caractérisée en ce que le réservoir est réalisé d'une seule pièce avec la gaine tubulaire, leurs espaces intérieurs se trouvant en communication et l'aiguille creuse dans la position de travail précitée se trouvant en communication étanche par la gaine tubulaire (14; 24) avec le réservoir (11; 21) de la seringue.

2. Seringue suivant la revendication 1, caractérisée en ce que le porte-aiguille (18.1; 28.1) avec sa région terminale (18.3; 28.3) d'où sort l'aiguille (18; 28) peut, dans la position de travail précitée avec l'aiguille (18; 28) sortie de la gaine tubulaire (14; 24), être couplé de manière amovible sous une légère pression avec un siège (14.2; 24.2) usiné dans une partie d'extrémité axiale (14.1; 24.1) de la gaine tubulaire et en ce qu'il présente des moyens (18.7; 28.7) d'étanchéité à l'égard de la paroi de l'espace creux de la gaine tubulaire, pour ainsi assurer une position de travail stable de l'aiguille et en même temps former dans la gaine tubulaire un espace hermétique, ainsi que pour réaliser une communication étanche entre le trou de l'aiguille et le réservoir (11; 21) à travers le porte-aiguille et par un trou de communication (17; 27) qui débouche dans l'espace fermé précité dans la gaine tubulaire.

3. Seringue suivant la revendication 2, caractérisée en ce que le siège précité (14.2; 24.2) dans la gaine tubulaire (14; 24) s'élargit en direction de l'espace intérieur de la gaine tubulaire en formant une embouchure (14.3; 24.3) destinée au couplage étanche et sous légère pression d'une partie (18.2; 28.2) du porte-aiguille (18.1; 28.1), partie qui se trouve derrière la région d'extrémité (18.3; 28.3) d'où sort l'aiguille (18; 28), ce qui accroît la stabilité de la position de travail de l'aiguille et l'efficacité de l'étanchéité.

4. Seringue suivant l'une ou l'autre des revendications précédentes, caractérisée en ce que le porte-aiguille (18.1) présente des moyens d'étanchéité (18.7) pour assurer l'étanchéité du mouvement de glissement le long de la totalité de l'espace intérieur axial de la gaine tubulaire (14), grâce auxquels un liquide peut être aspiré et/ou injecté au moyen de la seringue (10) même dans la position de sécurité et de protection de l'aiguille (18) dans la gaine tubulaire (14).

5. Seringue suivant l'une ou l'autre des revendications précédentes, caractérisée en ce que le porte-aiguille (18.1) peut coulisser axialement dans la gaine tubulaire (14) en même temps qu'une tige (19) qui est fixée sur lui, la tige sortant à l'extérieur de la gaine tubulaire et pouvant être actionnée manuellement.

6. Seringue suivant la revendication 5, caractérisée en ce que la tige (19) présente un point de rupture (19.2) qui se trouve à l'extérieur de la gaine tubulaire lorsque l'aiguille (18) est en position de sécurité et de protection dans la gaine tubulaire (14).

7. Seringue suivant l'une ou l'autre des revendications précédentes 1 à 5, caractérisée en ce que le porte-aiguille (28.1) est, lors du mouvement axial destiné à faire sortir l'aiguille (28) hors de la gaine tubulaire (24), guidé au moyen d'un patin (30) actionnable manuellement contre l'action d'un ressort de rappel (29), le retour automatique du porte-aiguille avec l'aiguille dans la gaine tubulaire étant provoqué par le ressort de rappel après la séparation du porte-aiguille hors du couplage étanche par rapport à la gaine tubulaire en position de travail.

8. Seringue suivant l'une ou l'autre des revendications précédentes, caractérisée en ce que la gaine tubulaire (14; 24) présente un ajutage dans une région d'extrémité (14.1; 24.1), une aiguille d' injection conventionnelle avec un porte-aiguille normalisé pouvant être posée sur cet ajutage pour effectuer des opérations d'injection et/ou d'aspiration avec la même seringue.

9. Seringue suivant l'une ou l'autre des revendications précédentes, caractérisée en ce que le gaine tubulaire (14) présente, à proximité de sa région d'extrémité axiale (14.5) qui est opposée à la région d'extrémité (14.1) munie de l'ouverture pour la sortie de l'aiguille (18), une embouchure plongeante (14.6) qui s'élargit en direction de l'espace intérieur de la gaine tubulaire et dans laquelle le porte-aiguille (18.1) est retenu à la fin de sa course de retour dans la gaine tubulaire.

10. Seringue suivant la revendication 1, caractérisée en ce que l'aiguille creuse peut aussi être mise en communication étanche avec le réservoir par au moins un trou radial, qui est prévu dans la paroi de l'aiguille d'injection et qui débouche dans une chambre étanche de la gaine tubulaire qui est en communication avec le même réservoir de la seringue.

11. Seringue suivant l'une ou l'autre des revendications précédentes, caractérisée en ce qu'un piston coulissant (12; 22) avec une tige de commande (13; 23) est disposé de manière étanche dans le réservoir (11; 21) de la seringue.

12. Seringue suivant l'une ou l'autre des revendications précédentes 1 à 10, caractérisée en ce qu'une capsule sous vide destinée à créer une dépression d'aspiration dans la seringue est placée dans le réservoir.

13. Seringue suivant l'une ou l'autre des revendications précédentes 1 à 10, caractérisée en ce que le réservoir de la seringue est constitué par une chambre avec une membrane élastique.

Fig. 1

Fig. 2

Fig. 3

Fig. 4

Fig. 5

Fig. 6

Fig. 7

Fig. 8

Fig. 9

Fig. 10

Fig. 11

Fig. 12

Fig. 13

Fig. 14